# EUROPÄISCHE PATENTSCHRIFT

(11) **EP 1 294 343 B1**
(45) Veröffentlichungstag und Bekanntmachung des Hinweises auf die Patenterteilung: **24.08.2005**
(21) Anmeldenummer: 01940575.2
(22) Anmeldetag: 15.06.2001
(51) Int. Cl.: A61K 7/06, A61K 7/13

(54) **MITTEL ZUR OXIDATIVEN FARBVERÄNDERUNG KERATINISCHER FASERN EIN VERDICKUNGSSYSTEM ENTHALTEND**
AGENTS CONTAINING A THICKENING SYSTEM,FOR THE OXIDATIVE COLOURING OF KERATIN FIBRES
AGENTS POUR LA COLORATION DES FIBRES KERATINIQUES COMPRENANT UN SYSTEME EPAISSISSANT

(30) Priorität: 26.06.2000 DE 10029981
(43) Veröffentlichungstag der Anmeldung: 26.03.2003
(73) Patentinhaber: Henkel Kommanditgesellschaft auf Aktien, 40589 Düsseldorf (DE)
(72) Erfinder: BERNECKER, Ullrich, 52393 Hürtgenwald (DE); HÖFFKES, Horst, 40595 Düsseldorf (DE); EHLERT, Manuela, 51379 Leverkusen (DE); SCHUMACHER, Ulrike, 40589 Düsseldorf (DE)
(86) Internationale Anmeldenummer: PCT/EP2001/006811
(87) Internationale Veröffentlichungsnummer: WO 2002/000177

(56) Entgegenhaltungen:
- EP-A- 0 241 707
- EP-A- 0 897 711
- WO-A-99/37278
- DE-C- 19 723 538
- DE-C- 19 815 341
- US-A- 4 685 931
- US-A- 5 393 305
- US-A1- 2001 002 254

## Beschreibung

Gegenstand der vorliegenden Anmeldung sind Mittel mit einem speziellen Verdickungssystem sowie ein Verfahren zur Verdickung oxidativer Systeme.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Ferner werden häufig aufhellende Verfahren, die sogenannten Blondierverfahren, angewendet. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare - insbesondere für die Strähnchenapplikation -werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Üblicherweise enthalten derartige Anwendungszubereitungen ein spezielles Verdickungssystem, um einem Abtropfen der Zubereitung von den Fasern entgegenzuwirken. So werden beispielsweise in der EP-A2-640 334 und der EP-A2-640 335 jeweils gelförmige Haarfärbemittel beschrieben, die Copolymere aus Acrylsäure und Acrylsäureestern als Verdickungsmittel in der Oxidationsmittelzubereitung enthalten. Um eine ausreichende Verdickungswirkung zu erzielen, müssen diese Polymere aber in einer relativ hohen Konzentration eingesetzt werden. Derartige Konzentrationen bedingen häufig Probleme bei der Herstellung der Färbemittel, da Polymerrückstände in den Produktionskesseln aushärten. Als Folge dieses Prozesses müssen die Kessel häufig aufwendig gereinigt werden.

Daher bestand die Aufgabe ein Verdickungsmittelsystem zu entwickeln, das
- in oxidativen Mitteln stabil eingesetzt werden kann,
- die oben genannten Nachteile in der Produktion vermeidet und
- eine ausreichende Verdickungsleistung aufweist.

Es wurde nun überraschenderweise gefunden, daß diese Anforderungen durch den Einsatz einer speziellen Kombination von Verdickungsmitteln in hohem Maße erfüllt werden.

Ein erster Gegenstand der vorliegenden Erfindung ist daher ein Mittel zur oxidativen Farbveränderung keratinischer Fasern, das ein Verdickungsmittelsystem aus mindestens einem Homo- oder Copolymeren (A), das gebildet wird aus einem Monomerengemisch, das mindestens ein ethylenisch ungesättigtes Monomer der allgemeinen Formel (I)

CHR⁴=CR¹R² (I)

enthält, wobei R¹ausgewählt ist aus Y(CH₂CH₂O)ₓ-R³ und COOH,
Y ausgewählt ist aus C(=O)O, C(=O)NH und CH₂O,
x eine Zahl von 1 bis 100, vorzugsweise von 10 bis 50 ist,
R³ steht für einen C₁- bis C₃₀- Alkylrest, vorzugsweise einen C₈- bis C₃₀-Alkylrest,
R² ausgewählt ist aus Wasserstoff, C₁- bis C₃₀-Alkyl und CH₂-R¹,
R⁴ ausgewählt ist aus Wasserstoff oder einer C₁- bis C₄-Alkylgruppe,
unter der Maßgabe, daß mindestens einer der Reste R¹ und R² die Gruppe Y-(CH₂CH₂O)ₓ-R³ enthält, und
mindestens einem Homo- oder Copolymeren (B), das gebildet wird aus einem Monomergemisch von ethylenisch ungesättigten Säuren und/oder deren einfachen C₁- bis C₆-Alkylestern, enthält, dadurch gekennzeichnet, daß es unmittelbar von der Anwendung auf den Fasern durch Mischung einer ersten, sauer eingestellten Komponente und einer zweiten, alkalisch eingestellten Komponente erhalten wird und das Homo-oder Copolymere (A) in der sauer eingestellten Komponente enthalten ist. Mithilfe des erfindungsgemäßen Verdickungssystems kann eine ausreichende Verdickung bereits mit einer geringeren Menge an verdickenden Polymeren erreicht werden. Ferner sind erfindungsgemäß verdickte Zubereitungen auch deutlich stabiler bei der Einwirkung von Scherkräften, wie beispielsweise Schütteln der Applikatorflasche.

Unter keratinischen Fasern werden erfindungsgemäß Pelze, Wolle, Federn und insbesondere menschliche Haare verstanden.

Sowohl die ethylenisch ungesättigten Monomere der allgemeinen Formel (I) als auch die Monomereinheiten der Komponente (B) sind bevorzugter Weise ausgewählt aus der Gruppe gebildet von der Acrylsäure, der Methacrylsäure und/oder der Itaconsäure sowie deren Derivaten.

Vorzugsweise ist die Komponente (A) ein Copolymer, welches gebildet ist aus mindestens einem ethylenisch ungesättigten Monomeren der allgemeinen Formel (I) sowie mindestens einem ethylenisch ungesättigten Monomeren der allgemeinen Formel (II)

CH₂=C(R⁶)COOR⁵ (II)

wobei R⁵ und R⁶ unabhängig voneinander ausgewählt sind aus Wasserstoff und einer Alkylgruppe mit 1 bis 30, vorzugsweise mit 1 bis 12, insbesondere bevorzugt mit 1 bis 4 Kohlenstoffatomen.

Bevorzugt ist weiterhin, daß die Gruppe Y der Formel (I) ausgewählt ist aus C(=O)O und CH₂O, daß R² ausgewählt ist aus Wasserstoff und Methyl oder daß es sich bei dem Monomer der Formel (I) um ein Itaconsäurederivat handelt. Ebenfalls bevorzugt ist, daß es sich bei dem Monomer der Formel (II) um Acrylsäure, Methacrylsäure oder einen ihrer C₁- bis C₄-Alkylester handelt.

Geeignete Copolymere sind beispielsweise Acryl- oder Methacrylsäure/Acryl- oder Methacrylsäurepolyethoxyalkylester-Copolymere (INCI-Bezeichnung: Acrylates/Steareth- 20 Methacrylate Copolymer), wie sie von der Firma Rohm und Haas/USA unter den Bezeichungen Acrysol®-22, Acrysol® ICS oder Aculyn®-22 vertrieben werden und Acryl- oder Methacrylsäure/Polyethoxyalkylallylether Copolymere (INCI-Bezeichnung: Steareth-10 Allyl Ether/Acrylates Copolymer), wie sie von der Firma Allied Colloids/Großbritannien unter der Bezeichnung Salcare® SC 90 vertrieben werden und Acryl- oder Methacrylsäure/Itaconsäurepolyethoxyalkylester-Copolymere (INCI-Bezeichnungen: Acrylates/Steareth-20 Itaconate Copolymer und Acrylates/Ceteth-20 Itaconate Copolymer), wie sie von der Firma National Starch/USA unter den Bezeichnungen Structure® 2001 und Structure® 3001 vertrieben werden. Weiterhin ist das unter der Bezeichnung Synthalen® W2000 (INCI-Bezeichnung: Acrylates/ C12-24 Pareth-25 Acrylate Copolymer) vertriebene Produkt der Firma 3V Sigma ein bevorzugtes Polymer (A).

Erfindungsgemäß bevorzugte Komponenten (B) sind Verdickungsmittel, die aus zwei oder mehreren Monomeren, ausgewählt aus Acrylsäure, Methacrylsäure und ihren C₁- bis C₆-Alkylestem, gebildet werden und unter der INCI-Bezeichnung Acrylates Copolymer vertrieben werden. Insbesonders sind die unter den Handelsbezeichnungen Yodosol® (National Starch & Chemical Company), Structure® O (National Starch & Chemical Company), Salcare® SC81 (Allied Colloids, Ltd.), Rohagit® S (Rohm GmbH Chemische Fabrik), Luviflex® Soft (BASF Corporation), Luvimer® (BASF Corporation) und Aculyn® 33 Polymer (Rohm and Haas Company, Inc.) vertriebenen Polymere bevorzugt. Ähnliche Produkte technischer Reinheit werden auch von der Firma BASF unter der Bezeichnung Latekoll® vertrieben.

Die Acryl- oder Methacrylsäuregruppen sind in den eingesetzten Polymeren (A) und (B) vorzugsweise durch organische oder anorganische Basen, insbesondere durch Ammoniak oder primäre oder sekundäre Amine, z.B. durch Aminomethylpropanol (AMP) neutralisiert. Das erfindungsgemäße Verdickungsmittelsystem ist in dem Haarbehandlungsmittel in einer Menge von 0,01 bis 10, vorzugsweise in einer Menge von 0,05 bis 4 Gew.-% enthalten.

Die erfindungsgemäß verdickten Mittel werden unmittelbar vor der Anwendung auf den Fasern durch Mischung einer ersten, alkalisch eingestellten, Komponente und einer zweiten, sauer eingestellten, Komponente erhalten.

Hinsichtlich der erzielbaren oxidativen Farbveränderung hat es sich als bevorzugt erwiesen, wenn die zweite, sauer eingestellte, Komponente mindestens ein Oxidationsmittel enthält. Als Oxidationsmittel kommen bevorzugterweise Persulfate, Chlorite und insbesondere Wasserstoffperoxid oder dessen Anlagerungsprodukte an Harnstoff, Melamin sowie Natriumborat in Frage. Als erfindungsgemäß besonders geeignet haben sich wäßrige Wasserstoffperoxidlösungen erwiesen.

Ferner hat es sich hinsichtlich der verdickenden Wirkung als vorteilhaft erwiesen, wenn das Homo- oder Copolymere (A), das gebildet wird aus einem Monomerengemisch, das mindestens ein ethylenisch ungesättigtes Monomer der allgemeinen Formel (I) enthält, in der sauer eingestellten Komponente enthalten ist. Eine besonders gute Verdickungsleistung kann erzielt werden, wenn das gesamte Verdickungssystem in der sauer eingestellten Komponente enthalten ist.

Im Rahmen der vorliegenden Erfindung haben sich Anwendungszubereitungen als besonders vorteilhaft erwiesen, die mithilfe der erfindungsgemäßen Verdickungssystems eine Viskosität von 1000 bis 7000 mPas aufweisen. Ganz besonders gute Anwendungseigenschaften treten bei Mitteln auf, die bei der Anwendung eine Viskosität von 3000 bis 6000 mPas aufweisen. Die Viskosität der Anwendungszubereitung wird erfindungsgemäß nach einer Standzeit von 1 Minute bei Raumtemperatur (20 °C) mit Hilfe eines Brookfield RVF Viskosimeters (Spindel 4, 20 U/min, 20°C) bestimmt.

### Oxidative Haarfärbung

In einer ersten bevorzugten Ausführungsform der vorliegenden Erfindung handelt es sich bei der oxidativen Farbveränderung keratinischer Fasern um eine oxidative Haarfärbung.

Oxidative Haarfärbemittel können als Farbstoffvorprodukte
- Oxidationsfarbstoffvorprodukte vom Entwickler- und Kupplertyp und/oder
- Farbstoffvorprodukte vom Indolin- und Indoltyp
enthalten.

Die Entwicklerkomponenten bilden unter dem Einfluß von Oxidationsmitteln, gegebenenfalls mit Hilfe spezieller Enzyme oder Metallionen als Katalysator, untereinander oder unter Kupplung mit einer oder mehreren Kupplerkomponenten die eigentlichen Farbstoffe aus.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 B1 bzw. WO 94/08970 A1 wie z.B. 4,5-Diamino-1-(2'-hydroxyethyl)-pyrazol.

Erfindungsgemäß besonders bevorzugt sind die Entwicklerkomponenten p-Phenylendiamin, p-Toluylendiamin, p-Aminophenol, 1-(2'-Hydroxyethyl)-2,5-diaminobenzol, N,N-Bis-(2-hydroxyethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4,5-Diamino-1-(2'-hydroxyethyl)-pyrazol.

Erfindungsgemäß bevorzugte Kupplerkomponenten sind
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol und
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Ferner können die oxidativen Färbemittel zur weiteren Nuancierung verschiedene direktziehende Farbstoffe enthalten.

Weitere in oxidativen Färbemitteln enthaltene Farbstoffvorprodukte können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Die Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1;2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.%, bezogen auf das gesamte Färbemittel.

Weiterhin können die Färbezubereitungen auch in der Natur vorkommende Farbstoffe wie beispielsweise Henna rot, Henna neutral, Henna schwarz, Kamillenblüte, Sandelholz, schwarzer Tee, Faulbaumrinde, Salbei, Blauholz, Krappwurzel, Catechu, Sedre und Alkannawurzel enthalten.

Das anwendungsbereite Haarfärbemittel ist üblicherweise alkalisch, d. h. auf pH-Werte im Bereich von etwa 7 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

### Blondiermittel

In einer zweiten bevorzugten Ausführungsform handelt es sich bei der oxidativen Farbveränderung keratinischer Fasern um eine Blondierung.

Im Rahmen eines derartigen Blondierverfahrens wird die Anwendungszubereitung durch Mischung eines sogenannten Blondiermittels mit einer Wasserstoffperoxidzubereitung erhalten. Die Blondiermittel können zusätzlich feste Anlagerungsverbindung von Wasserstoffperoxid an anorganische oder organische Verbindungen, wie beispielsweise Natriumborat, Natriumcarbonat, Natriumcarbamid, Polyvinylpyrrolidon, Harnstoff und Melamin, enthalten.

Die Blondierwirkung kann durch sogenannte "Booster" gesteigert werden. Dies sind in der Regel feste Peroxoverbindung, die keine Anlagerungsprodukte von Wasserstoffperoxid an andere Komponenten darstellen. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat und Peroxide wie Bariumperoxid. Diese Peroxoverbindungenkönnen auch in Kombination eingesetzt werden. Besonders bevorzugt sind die Peroxidisulfate, insbesondere Ammoniumperoxidisulfat.

Die Peroxoverbindungen sind in den erfindungsgemäßen Blondiermitteln bevorzugt in Mengen von 5-30 Gew.-%, insbesondere in Mengen von 8-20 Gew.-%, enthalten.

Als weitere wichtige Komponente enthalten Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxide, -carbonate, -hydrogencarbonate, - hydroxycarbonate,
-silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen aus einem Metasilikat und einem Hydroxycarbonat bevorzugt sein.

Blondiermittel enthalten Alkalisierungsmittel bevorzugt in Mengen von 5-25 Gew.-%, insbesondere 10-20 Gew.-%.

Für die Anwendung der Mittel auf dem Haar werden die Blondiermittel unmittelbar vor dem Auftragen mit einer Wasserstoffperoxidlösung vermischt. Die Konzentration dieser Wasserstoffperoxidlösung wird einerseits von den gesetzlichen Vorgaben und andererseits von dem gewünschten Effekt bestimmt; in der Regel werden 6- bis 12-prozentige sauer eingestellte Lösungen in Wasser verwendet. Die Mengenverhältnisse von Blondiermittel und Wasserstoffperoxidlösung liegen üblicherweise im Bereich 1:1 bis 1:2, wobei ein Überschuß an Wasserstoffperoxidlösung insbesondere dann gewählt wird, wenn keine zu ausgeprägte Blondierwirkung erwünscht ist.

Sollen die Fasern neben einer starken Aufhellung gleichzeitig auch in einer bestimmten Nuance gefärbt werden, so können die Blondiermittel weiterhin auch Farbstoffvorprodukte und / oder direktziehende Farbstoffe enthalten. In diesem Zusammenhang sei auf die obigen Ausführungen verwiesen.

### Anwendungsbedingungen

Sowohl bei der oxidativen Haarfärbung als auch bei der Blondierung ist es möglich, die Oxidation mit Hilfe von Enzymen zu beschleunigen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Substraten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Ferner kann die Variation der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺,Ru³⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Die Anwendungstemperaturen können im Rahmen der erfindungsgemäßen oxidativen Farbveränderung in einem Bereich zwischen 15 und 40 °C, bevorzugt bei der Temperatur der Kopfhaut, liegen. Die Einwirkungszeit der Anwendungszubereitung auf den Fasern beträgt bevorzugt zwischen 5 und 45, insbesondere 15 bis 30, Minuten. Im Anschluß an diese Einwirkungszeit werden die Fasern üblicherweise mit Wasser und gegebenenfalls einem geeigneten Shampoo gründlich ausgewaschen.

Das Nachwaschen mit einem Shampoo entfällt, wenn ein stark tensidhaltiger Träger, z. B. ein Färbeshampoo, verwendet wurde.

### Tenside

In einer weiteren bevorzugten Ausführungsform enthalten die erfindungsgemäßen Mittel mindestens ein Tensid. Unter dem Begriff Tenside werden erfindungsgemäß oberflächenaktive Substanzen verstanden, die nicht-ionischer, anionischer, kationischer zwitterionischer oder amphoterer Natur sein können.

Nichtionische Tenside enthalten als hydrophile Gruppe beispielsweise eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (III)

   R⁷CO-(OCH₂CHR⁸)_{w}OR⁹ (III),

   in der R⁷CO für einen linearen oder verzweigten, gesättigten und/oder ungesättigten Acylrest mit 6 bis 22 Kohlenstoffatomen, R⁸ für Wasserstoff oder Methyl, R⁹ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-A-197 38 866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)x-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (IV), 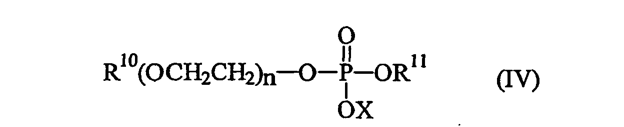 in der R¹⁰ bevorzugt für einen aliphatischen Kohlenwasserstoffrest mit 8 bis 30 Kohlenstoffatomen, R¹¹ für Wasserstoff, einen Rest (CH₂CH₂O)ₙR¹⁰ oder X, n für Zahlen von 1 bis 10 und X für Wasserstoff, ein Alkali- oder Erdalkalimetall oder NR¹²R¹³R¹⁴R¹⁵, mit R¹² bis R¹⁵ unabhängig voneinander stehend für einen C₁ bis C₄ - Kohlenwasserstoffrest, steht,
- sulfatierte Fettsäurealkylenglykolester der Formel (V)

   R¹⁶CO(AlkO)ₙSO₃M (V)

   in der R¹⁶CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-A-197 36 906 beschrieben sind,
- Monoglyceridsulfate und Monoglyceridethersulfate der Formel (VI) wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind. 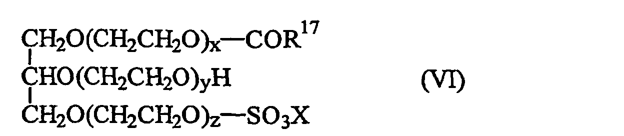 in der R¹⁷CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (VI) eingesetzt, in der R³⁶CO für einen linearen Acylrest mit 8 bis 18 Kohlenstoffatomen steht

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Die genannten Tenside werden in Mengen von 0,1 - 45 Gew.%, bevorzugt 1 - 30 Gew.-% und ganz besonders bevorzugt von 1 - 15 Gew.-%, bezogen auf das gesamte Mittel, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.%, bezogen auf das gesamte Mittel, enthalten. Mengen von 0,1 bis 5 Gew.-% sind besonders bevorzugt.

Unter den genannten Tensid-Typen können die Tenside, welche kein Ethylenoxid und/oder Propylenoxid im Molekül enthalten, ganz besonders bevorzugt sein.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- weitere Verdickungsmittel wie Agar-Agar, Guar-Gum, Alginate, Xanthan-Gum, Gummi arabicum, Karaya-Gummi, Johannisbrotkernmehl, Leinsamengummen, Dextrane, CelluloseDerivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdom, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der vorliegenden Erfindung ist ein Verfahren zur Verdickung oxidativer Systeme, die unmittelbar vor der Auwendung auf den Fasern durch Mischung einer ersten, sauer eingestellten Komponente und einer zweiten, alkalisch eingestellten Komponente erhalten werden, dadurch gekennzeichnet, daß ein Verdickungsmittelsystem aus mindestens einem Homo- oder Copolymeren (A), das gebildet wird aus einem Monomerengemisch, das mindestens ein ethylenisch ungesättigtes Monomer der allgemeinen Formel (I)

CHR⁴=CR¹R² (I)

enthält, wobei R¹ausgewählt ist aus Y-(CH₂CH₂O)ₓ-R³ und COOH,
Y ausgewählt ist aus C(=O)O, C(=O)NH und CH₂O,
x eine Zahl von 1 bis 100, vorzugsweise von 10 bis 50 ist,
R³ steht für einen C₁- bis C₃₀- Alkylrest, vorzugsweise einen C₈- bis C₃₀-Alkylrest,
R² ausgewählt ist aus H, C₁- bis C₃₀-Alkyl und CH₂-R¹,
R⁴ ausgewählt ist aus Wasserstoff oder einer C₁- bis C₄-Alkylgruppe,
unter der Maßgabe, daß mindestens einer der Reste R¹ und R² die Gruppe Y-(CH₂CH₂O)ₓ-R³ enthält, und
mindestens einem Homo- oder Copolymeren (B), das gebildet wird aus einem Monomergemisch von ethylenisch ungesättigten Säuren und/oder deren einfachen C₁- bis C₆-Alkylestern, wobei das Homo- oder Copolymere (A) in der sauer eingestellten Komponente enthalten ist, verwendet wird.

### Beispiele

Die folgenden Mengenangaben sind soweit nichts anderes vermerkt ist in Gewichtsprozent.

Es wurde die folgenden Oxidationsmittelzubereitungen erstellt:

| Rohstoff | Vergleichs-rezeptur A | Vergleichs-rezeptur B | Vergleichs-rezeptur C | Beispiel E |
|---|---|---|---|---|
| Texapon® NSO¹ | 2,0 | 2,0 | 2,0 | 2,0 |
| Dipicolinsäure | 0,1 | 0,1 | 0,1 | 0,1 |
| Na-pyrophosphat | 0,03 | 0,03 | 0,03 | 0,03 |
| Turpinal® SL² | 1,5 | 1,5 | 1,5 | 1,5 |
| Dow Corning® DB 110 A³ | 0,07 | 0,07 | 0,07 | 0,07 |
| Aculyn® 33⁴ | 12,0 | 4,0 | --- | 4,0 |
| Structure® 2001⁵ | --- | --- | 0,5 | 0,5 |
| Wasserstoffperoxid⁶ | 22,4 | 22,4 | 22,4 | 22,4 |
| Ammoniak, 25%ig | 0,65 | 0,65 | 0,65 | 0,65 |
| Wasser | ad 100 | ad 100 | ad 100 | ad 100 |

| | | | | |
|---|---|---|---|---|
| ¹ Laurylethersulfat-Natrium-Salz (INCI-Bezeichnung: Sodium Laureth Sulfate, ca. 26,5% Aktivsubstanz) (Cognis) | | | | |
| ² 1-Hydroxyethan-1,1-diphosphonsäure (INCI-Bezeichnung: Etidronic Acid) (Cognis) | | | | |
| ³ nichtionogene Silicon-Emulsion (INCI-Bezeichnung: Dimethicone, ca. 10% Aktivsubstanz) (Dow Corning) | | | | |
| ⁴ Säure-enthaltendes, vernetztes Acrylcopolymer (INCI-Bezeichnung: Acrylates Copolymer, ca. 28% Aktivsubstanz) (Rohm&Haas) | | | | |
| ⁵ Acryl-Copolymer (INCI-Bezeichnung: Acrylates/Steareth-20 Itaconate Copolymer, ca. 29% Aktivsubstanz) | | | | |
| ⁶ 50%ige wäßrige Lösung | | | | |

Ferner wurde die folgende Blondiercreme erstellt:

| | |
|---|---|
| C₁₆₋₁₈-Fettalkohol | 10,0 |
| C₁₂₋₁₈-Fettalkohol | 2,0 |
| Texapon® NSO | 26,5 |
| Ammoniumsulfat | 1,0 |
| Natronwasserglas | 0,5 |
| Ammoniak, 25%ig | 7,6 |
| Gluadin® W40⁷ | 0,35 |
| Parfüm | q.s. |
| Wasser | ad 100 |

| | |
|---|---|
| ⁷ Weizenproteinhydrolysat (INCI-Bezeichnung: Hydrolyzed Wheat Protein, Sodium Benzoate, Phenoxyethanol, Methylparaben, Propylparaben, ca. 40% Aktivsubstanz) (Cognis) | |

Es wurden äquivalente Mengen der Oxidationsmittelzubereitung und der Blondiercreme vermischt. Die Viskositäten der resultierenden Anwendungszubereitungen wurden mit eine Brookfield RVF Viskosimeter (Spindel 4, 20U/min, 20°C) nach einer Standzeit von 1 Minute bei Raumtemperatur bestimmt. Die Meßwerte sind der Tabelle II zu entnehmen.

**Tabelle II**

| Rezeptur | Viskosität der Anwendungszubereitung in mPas |
|---|---|
| A | 4900 |
| B | 1600 |
| C | 1450 |
| E (Erfindungsgemäß) | 3300 |

Während die Viskosität der Rezeptur C nach Schütteln der Applikatorflasche schnell wieder abnimmt, ist die Viskosität der Rezeptur E stabil.

Mithilfe des erfindungsgemäßen Verdickungssystems (Rezeptur E) ist es möglich, mit einer geringeren Menge an verdickenden Polymeren als in Versuch A eine zufriedenstellende Viskosität der Anwendungszubereitung zu erreichen. Die Verdickungsleistung der erfindungsgemäßen Kombination ist deutlich höher, als es die Einzelkomponenten (Rezeptur B und Rezeptur C) erwarten lassen.

## Patentansprüche

1. Mittel zur oxidativen Farbveränderung keratinischer Fasern, enthaltend ein Verdickungsmittelsystem aus
mindestens einem Homo- oder Copolymeren (A), das gebildet wird aus einem Monomerengemisch, das mindestens ein ethylenisch ungesättigtes Monomer der allgemeinen Formel (I)
CHR⁴=CR¹R² (I)
enthält, wobei R¹ausgewählt ist aus Y-(CH₂CH₂O)ₓ-R³ und COOH,
Y ausgewählt ist aus C(=O)O, C(=O)NH und CH₂O,
x eine Zahl von 1 bis 100, vorzugsweise von 10 bis 50 ist,
R³ steht für einen C₁- bis C₃₀- Alkylrest, vorzugsweise einen C₈- bis C₃₀-Alkylrest,
R² ausgewählt ist aus H, C₁- bis C₃₀-Alkyl und CH₂-R¹,
R⁴ ausgewählt ist aus Wasserstoff oder einer C₁- bis C₄-Alkylgruppe,
unter der Maßgabe, dass mindestens einer der Reste R¹ und R² die Gruppe Y-(CH₂CH₂O)ₓ-R³ darstellt, und
mindestens einem Homo- oder Copolymeren (B), das gebildet wird aus einem Monomergemisch von ethylenisch ungesättigten Säuren und/oder deren einfachen C₁-bis C₆-Alkylestern,
enthält, **dadurch gekennzeichnet, dass** es unmittelbar vor der Anwendung auf den Fasern durch Mischung einer ersten, sauer eingestellten. Komponente und einer zweiten, alkalisch eingestellten Komponente erhalten wird und das Homo- oder Copolymere (A) in der sauer eingestellten Komponente enthalten ist.

2. Mittel nach Anspruch 1, **dadurch gekennzeichnet, dass** die sauer eingestellte Komponente mindestens ein Oxidationsmittel enthält.

3. Mittel nach Anspruch 2, **dadurch gekennzeichnet, dass** das Oxidationsmittel Wasserstoffperoxid ist.

4. Mittel nach einem der Ansprüche 1 bis 3, **dadurch gekennzeichnet, dass** das gesamte Verdickungssystem in der sauer eingestellten Komponente enthalten ist.

5. Mittel nach einem der Ansprüche 1 bis 4, **dadurch gekennzeichnet, dass** die Viskosität der Anwendungszubereitung 1000 bis 7000 mPas beträgt.

6. Mittel nach Anspruch 5, **dadurch gekennzeichnet, dass** die Viskosität der Anwendungszubereitung 3000 bis 6000 mPas beträgt.

7. Mittel nach einem der Ansprüche 1 bis 6, **dadurch gekennzeichnet, dass** es mindestens ein Oxidationsfarbstoffvorprodukt vom Typ der Entwicklerkomponenten enthält.

8. Mittel nach einem der Ansprüche 1 bis 7, **dadurch gekennzeichnet, dass** es mindestens ein Farbstoffvorprodukt vom Typ der Indole und/oder Indoline enthält.

9. Mittel nach einem der Ansprüche 1 bis 8, **dadurch gekennzeichnet, dass** es mindestens eine Peroxoverbindung enthält.

10. Verfahren zur Verdickung oxidativer Systeme, die unmittelbar vor der Anwendung auf den Fasern durch Mischung einer ersten, sauer eingestellten, Komponente und einer zweiten, alkalisch eingestellten Komponente erhalten werden, **dadurch gekennzeichnet, dass** ein Verdickungsmittelsystem aus mindestens einem Homo- oder Copolymeren (A), das gebildet wird aus einem Monomerengemisch, das mindestens ein ethylenisch ungesättigtes Monomer der allgemeinen Formel (I)
CHR⁴=CR¹R² (I)
enthält, wobei R¹ausgewählt ist aus Y-(CH₂CH₂O)ₓ-R³ und COOH,
Y ausgewählt ist aus C(=O)O, C(=O)NH und CH₂O,
x eine Zahl von 1 bis 100, vorzugsweise von 10 bis 50 ist,
R³ steht für einen C₁- bis C₃₀- Alkylrest, vorzugsweise einen C₈- bis C₃₀-Alkylrest,
R² ausgewählt ist aus H, C₁- bis C₃₀-Alkyl und CH₂-R¹,
R⁴ ausgewählt ist aus Wasserstoff oder einer C₁- bis C₄-Alkylgruppe,
unter der Maßgabe, dass mindestens einer der Reste R¹ und R² die Gruppe Y-(CH₂CH₂O)ₓ-R³ darstellt, und
mindestens einem Homo- oder Copolymeren (B), das gebildet wird aus einem Monomergemisch von ethylenisch ungesättigten Säuren und/oder deren einfachen C₁-bis C₆-Alkylestern, wobei das Homo- oder Copolymere (A) in der sauer eingestellten Komponente enthalten ist,
verwendet wird.

## Claims

1. Agent for the oxidative colour-changing of keratin fibres, comprising a thickener system of at least one homopolymer or copolymer (A) which is formed from a monomer mixture which comprises at least one ethylenically unsaturated monomer of the general formula (I)
CHR⁴=CR¹R² (I)
where
R¹ is chosen from Y-(CH₂CH₂O)ₓ-R³ and COOH,
Y is chosen from C(=O)O, C(=O)NH and CH₂O,
x is a number from 1 to 100, preferably from 10 to 50,
R³ is a C₁- to C₃₀- alkyl radical, preferably a C₈- to C₃₀- alkyl radical,
R² is chosen from H, C₁- to C₃₀- alkyl and CH₂-R¹,
R⁴ is chosen from hydrogen or a C₁- to C₄- alkyl group,
with the proviso that at least one of the radicals R¹ and R² is the group Y-(CH₂CH₂O)ₓ-R³, and
at least one homopolymer or copolymer (B) which is formed from a monomer mixture of ethylenically unsaturated acids and/or simple C₁- to C₆- alkyl esters thereof,
**characterized in that** it is obtained directly prior to application to the fibres by mixing a first component which has been rendered acidic and a second component which has been rendered alkaline, and the homopolymer and copolymer (A) is present in the component which has been rendered acidic.

2. Agent according to Claim 1, **characterized in that** the component which has been rendered acidic comprises at least one oxidizing agent.

3. Agent according to Claim 2, **characterized in that** the oxidizing agent is hydrogen peroxide.

4. Agent according to one of Claims 1 to 3,
**characterized in that** the entire thickening system is present in the component which has been rendered acidic.

5. Agent according to one of Claims 1 to 4,
**characterized in that** the viscosity of the application preparation is 1000 to 7000 mPas.

6. Agent according to Claim 5, **characterized in that** the viscosity of the application preparation is 3000 to 6000 mPas.

7. Agent according to one of Claims 1 to 6,
**characterized in that** it comprises at least one oxidation dye precursor of the developer component type.

8. Agent according to one of Claims 1 to 7,
**characterized in that** it comprises at least one dye precursor of the indole and/or indoline type.

9. Agent according to one of Claims 1 to 8,
**characterized in that** it comprises at least one peroxo compound.

10. Method of thickening oxidative systems which are obtained directly prior to application to the fibres by mixing a first component which has been rendered acidic, and a second component which has been rendered alkaline, **characterized in that** a thickener system of
at least one homopolymer or copolymer (A) which is formed from a monomer mixture which comprises at least one ethylenically unsaturated monomer of the general formula (I)
CHR⁴=CR¹R² (I)
where
R¹ is chosen from Y-(CH₂CH₂O)ₓ-R³ and COOH,
Y is chosen from C(=O)O, C(=O)NH and CH₂O,
x is a number from 1 to 100, preferably from 10 to 50,
R³ is a C₁- to C₃₀- alkyl radical, preferably a C₈- to C₃₀- alkyl radical,
R² is chosen from H, C₁- to C₃₀- alkyl and CH₂-R¹,
R⁴ is chosen from hydrogen or a C₁- to C₄- alkyl group,
with the proviso that at least one of the radicals R¹ and R² is the group Y-(CH₂CH₂O)ₓ-R³, and
at least one homopolymer or copolymer (B) which is formed from a monomer mixture of ethylenically unsaturated acids and/or simple C₁- to C₆- alkyl esters thereof, where the homopolymer or copolymer (A) is present in the component which has been rendered acidic, is used.

## Revendications

1. Agent pour la modification, par oxydation, de la couleur de fibres kératiniques, contenant un système épaississant constitué de
au moins un homo- ou copolymère (A), qui est formé d'un mélange de monomères contenant au moins un monomère éthyléniquement insaturé de formule générale (I)
CHR⁴=CR¹R² (I)
dans laquelle
R¹ est choisi parmi Y-(CH₂CH₂O)ₓ-R³ et COOH,
Y est choisi parmi C(=O)O, C(=O)NH et CH₂O,
x est un nombre allant de 1 à 100, de préférence de 10 à 50,
R³ représente un radical alkyle en C₁ à C₃₀, de préférence un radical alkyle en C₈ à C₃₀,
R² est choisi parmi H, alkyle en C₁ à C₃₀ et CH₂-R¹,
R⁴ est choisi parmi l'hydrogène ou un groupe alkyle en C₁ à C₄,
sous réserve qu'au moins un des radicaux R¹ et R² représente le groupe Y-(CH₂CH₂O)ₓ-R³, et
au moins un homo- ou copolymère (B) qui est formé d'un mélange monomère d'acides éthyléniquement insaturés et/ou de leurs esters simples d'alkyle en C₁ à C₆,
**caractérisé en ce qu'**il est obtenu immédiatement avant l'application sur les fibres par mélange d'un premier composant, rendu acide, et d'un second composant, rendu alcalin, et **en ce que** l'homo- ou copolymère (A) est contenu dans le composant rendu acide.

2. Agent selon la revendication 1, **caractérisé en ce que** le composant rendu acide contient au moins un oxydant.

3. Agent selon la revendication 2, **caractérisé en ce que** l'oxydant est le peroxyde d'hydrogène.

4. Agent selon l'une des revendications 1 à 3 , **caractérisé en ce que** l'ensemble du système épaississant est contenu dans le composant rendu acide.

5. Agent selon l'une des revendications 1 à 4, **caractérisé en ce que** la viscosité de la préparation d'application est de 1000 à 7000 mPas.

6. Agent selon la revendication 5, **caractérisé en ce que** la viscosité de la préparation d'application s'élève à 3000 à 6000 mPas.

7. Agent selon l'une des revendications 1 à 6, **caractérisé en ce qu'**il contient au moins un précurseur de colorant d'oxydation du type des composants de développement.

8. Agent selon l'une des revendications 1 à 7, **caractérisé en ce qu'**il contient au moins un précurseur de colorant du type des indoles et/ou des indolines.

9. Agent selon l'une des revendications 1 à 8, **caractérisé en ce qu'**il contient au moins un composé peroxo.

10. Procédé pour épaissir les systèmes d'oxydation que l'on obtient immédiatement avant l'application sur les fibres par mélange d'un premier composant rendu acide et d'un second composant rendu alcalin, **caractérisé en ce qu'**on utilise un système épaississant constitué de au moins un homo- ou copolymère (A), qui est formé d'un mélange de monomères contenant au moins un monomère éthyléniquement insaturé de formule générale (I)
CHR⁴=CR¹R² (I)
dans laquelle R¹ est choisi parmi Y-(CH₂CH₂O)ₓ-R³ et COOH,
Y est choisi parmi C(=O)O, C(=O)NH et CH₂O,
x est un nombre allant de 1 à 100, de préférence de 10 à 50,
R³ représente un radical alkyle en C₁ à C₃₀, de préférence un radical alkyle en C₈ à C₃₀,
R² est choisi parmi H, alkyle en C₁ à C₃₀ et CH₂-R¹,
R⁴ est choisi parmi l'hydrogène ou un groupe alkyle en C₁ à C₄,
sous réserve qu'au moins un des radicaux R¹ et R² représente le groupe Y-(CH₂CH₂O)ₓ-R³, et
au moins un homo- ou copolymère (B) qui est formé d'un mélange monomère d'acides éthyléniquement insaturés et/ou de leurs esters simples d'alkyle en C₁ à C₆,
l'homo- ou copolymère (A) étant contenu dans le composant rendu acide.
